# EUROPEAN PATENT APPLICATION

(11) **EP 2 189 171 A1**
(43) Date of publication of application: **26.05.2010**
(21) Application number: 08169853.2
(22) Date of filing: 25.11.2008
(51) Int. Cl.: A61M 1/00, A61M 25/00

(54) **Mobile chest drainage unit, thoracic catheter, system comprising a mobile chest drainage unit and a thoracic catheter, and manufacturing method thereof**

(71) Applicant: Covidien AG, 8212 Neuhausen am Rheinfall (CH)
(72) Inventor: Daly, Paul, Tullamore, Co. Offaly (IE)
(74) Representative: Körber, Martin Hans

(57) **Abstract**

The present invention relates to a mobile chest drainage unit (I) connectable to a thoracic catheter (3) for suctioning air and/or fluid from a patient's body. The thoracic catheter (3) comprises at least one sensor (5) the measuring physiologic parameters, which are transmitted to the mobile chest drainage unit (1). In one embodiment, an electronic connection can be provided between the mobile chest drainage unit (1) and the thoracic catheter.

## Description

The present invention relates to a mobile chest drainage unit, to a thoracic catheter and to a system comprising a mobile chest drainage unit and a thoracic catheter. The present invention further relates to a method of manufacturing a mobile chest drainage unit and to a method of manufacturing a thoracic catheter.

The clinical need for chest drainage arises anytime the negative pressure in the pleural cavity is disrupted by the presence of air and/or fluid resulting in pulmonary compromise. The purpose of a chest drainage unit is to evacuate the air and/or fluid from the chest cavity to help re-establish normal intrathoracic pressure. This facilitates the re-expansion of the lungs to restore normal briefing dynamics. The need also arises following heart surgery to prevent the accumulation of fluid around the heart.

Patients with continual air or fluid leaks have a chest tube, also called a thoracic catheter, inserted. The distal end, which will be inside the patient's chest, has a number of drainage holes. A radiopaque line is provided at the distal end of the thoracic catheter, so that the last eyelet can be detected on a chest X-ray as intermittent breaks in the radiopaque line. Once the chest tube has been properly positioned and secured, the X-ray should be checked to ensure that all drainage holes are inside the chest wall.

The location of the chest tube depends on what is being drained. Free air in the pleural space rises, so the tube is placed above the second intercostal space at the mid-clavicular line. Pleural fluid gravitates to the most dependent point, so the tube is placed at the fourth to fifth intercostal space along the mid-axillary line. Mediastinal tubes placed to drain the pericardium after open-heart surgery are positioned directly under the sternum. Once the chest tube is in place, it is connected to a chest drainage unit.

A chest drainage unit (CDU) usually comprises a collection chamber for collecting drainage from the chest. The collection chamber is graduated and has a write-on surface to allow for easy measurement and recording of the time, date and amount of drainage.

Portable electronic chest drainage systems allow patients to ambulate in a hospital or to be discharged to their homes with active suction draining fluids and gases from bodily areas, including sites proximate to surgical procedures. The portable chest tube drainage system includes a vacuum chamber, a vacuum pump housing and a fluid reservoir. These components can be removable connected to the portable drainage system and are disposable. The vacuum pump housing includes a vacuum source which may comprise a small vacuum pump and one or more removable, replaceable and/or rechargeable batteries.

One example of a chest tube drainage system is shown in document WO 2007/024230 A1. This document further teaches that a variety of sensors can be provided within the chest tube drainage system for monitoring a variety of parameters.

It is therefore the objective problem of the present invention to improve the prior art.

This object is solved by the features of the independent claims.

The present invention relates to a mobile chest drainage unit connectable to a thoracic catheter for suctioning air and/or fluid from a patient's body, said thoracic catheter comprising at least one sensor for acquiring information regarding physiological parameters, and said mobile chest drainage unit comprising a receiving means for receiving the acquired information from the at least one sensor.

Advantageously, the mobile chest drainage unit further comprises a drain tube, wherein said drain tube comprises a CDU tube connector for connecting the drain tube to a corresponding catheter tube connector of the thoracic catheter.

In one embodiment said receiving means is adapted to wirelessly receive the acquired information, preferably to receive the acquired information via radiofrequency RF, Bluetooth or infrared IR

In a further embodiment said receiving means is electronically connectable to the at least one sensor by electronic connection means. Hereby, said electronic connection means comprises an electric CDU connector electronically connectable to an electric catheter connector, which is electronically connected to the at least one sensor.

The present invention further relates to a mobile chest drainage unit comprising a drain tube connectable to a thoracic catheter for suctioning air and/or fluid from a patients body, and electronic connection means for electronically connecting the mobile chest drainage unit to the thoracic catheter.

Advantageously, said drain tube comprises a drain tube connector for connecting the drain tube to a corresponding catheter tube connector of the thoracic catheter.

Preferably, said electronic connection means comprise an electric CDU connector attached to the drain tube and electronically connectable to a corresponding electric catheter connector of the thoracic catheter.

The present invention further relates to a mobile chest drainage unit electronically connectable to a thoracic catheter.

In a further aspect the present invention relates to a thoracic catheter connectable to a mobile chest drainage unit, comprising at least one sensor for acquiring information regarding physiological parameters, and a transmitting means for transmitting the acquired information to the mobile chest drainage unit.

Preferably, said at least one sensor is provided at the distal end of the thoracic catheter, which is adapted to be inserted into a patient's body.

Further, preferably said at least one sensor is adapted to acquire information regarding blood pressure, respiration rate, heart beat rate, temperature and/or pressure within the pleural cavity.

Advantageously, said transmitting means via a catheter conductor is electronically connected to the at least one sensor. In a preferred embodiment, said catheter conductor is a radiopaque line.

In one embodiment, said transmitting means is a fixed transmitter adapted to wirelessly transmit the acquired information, preferably to transmit the acquired information via radiofrequency RF, Bluetooth or infrared IR.

In a further embodiment said transmitting means comprises an electric catheter connector electronically connectable to a connectable transmitter adapted to wirelessly transmit the acquired information, preferably to transmit the acquired information via radiofrequency RF, Bluetooth or infrared IR.

In still a further embodiment said transmitting means comprises an electric catheter connector electronically connectable to a corresponding electric CDU connector, which is electronically connected to the mobile chest drainage unit.

The present invention further relates to a thoracic catheter connectable to a mobile chest drainage unit via a drain tube attached to the mobile chest drainage unit and via electronic connection means attached to the drain tube and electronically connected to the mobile chest drainage unit.

Preferably, the thoracic catheter comprises a catheter tube connector for connecting the thoracic catheter to a corresponding drain tube connector of the drain tube.

Further, preferably, the thoracic catheter comprises an electronic catheter connector electronically connectable to a corresponding electric CDU connector being electronically connected to the mobile chest drainage unit.

The present invention further relates to a thoracic catheter electronically connectable to a mobile chest drainage unit.

According to a further aspect the present invention relates to a system comprising a mobile chest drainage unit according to any of the preceding claims connected to a thoracic catheter according to any of the preceding claims.

According to still a further aspect the present invention relates to a mobile chest drainage unit comprising a thoracic catheter, said thoracic catheter having at least one sensor for acquiring information regarding physiological parameters, a drain tube connecting the thoracic catheter with the mobile drainage unit, and a conductor along the drain tube for electronically connecting the at least one sensor with the mobile chest drainage unit.

According to a further aspect the present invention relates to a method of manufacturing a mobile chest drainage unit according to any of the preceding claims.

According to a further aspect the present invention relates to a method of manufacturing a thoracic catheter according to any of the preceding claims.

The present invention will now be explained in more detail in the following description of preferred embodiments in relation to the enclosed drawings in which
fig. 1 shows an example of a mobile chest drainage unit connected to a patient's body,
fig. 2 shows a schematic drawing of the distal end of a thoracic catheter according to the present invention,
fig. 3 shows a schematic drawing of a mobile chest drainage unit and a thoracic catheter connectable to each other according to the present invention,
fig. 4 shows a first embodiment of a mobile chest drainage unit and of a thoracic catheter according to the present invention,
fig. 5 shows the first embodiment of a mobile chest drainage unit and a second embodiment of a thoracic catheter according to the present invention,
fig. 6 shows a second embodiment of a mobile chest drainage unit and the second embodiment of a thoracic catheter according to the present invention, and
fig. 7 shows a further embodiment of a mobile chest drainage unit with a thoracic catheter according to the present invention.

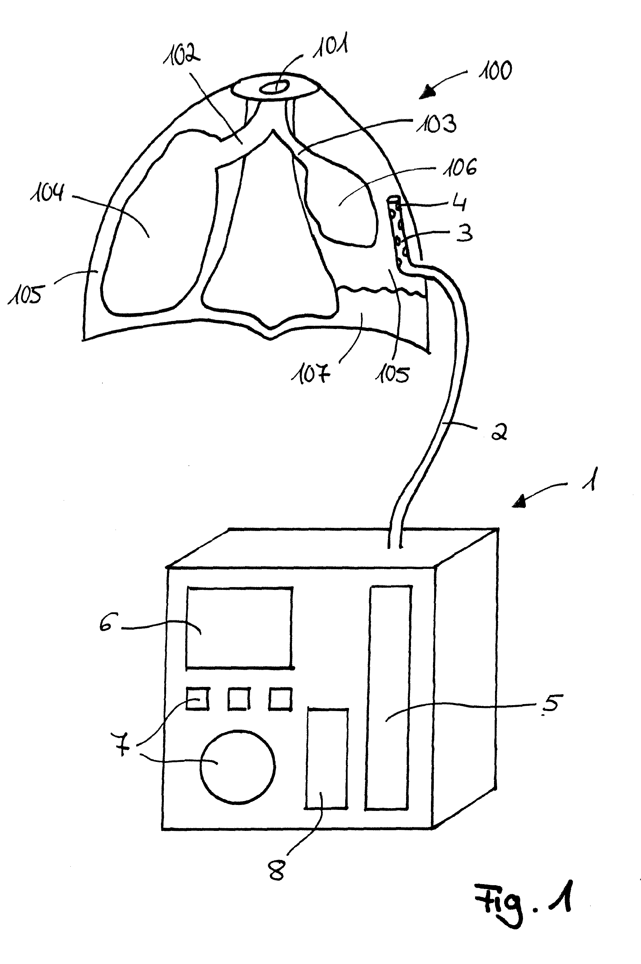

The present invention refers to a mobile chest drainage unit 1 used for draining air and/or fluid from a patient's body. An example of such a mobile chest drainage unit 1 is shown in fig. 1. The mobile chest drainage unit 1 usually comprises a collection chamber or fluid reservoir 5 and a vacuum chamber 8. Usually, a display 6 is provided, where for example actual parameters, e.g. actual negative pressure applied to the patient's body, or other parameters or settings can be shown. Advantageously, buttons 7 are further provided for enabling the physician and/or the nurse to regulate the mobile chest drainage unit 1 and to input information.

Attached to the mobile chest drainage unit 1 is a drain tube 2 which connects to the thoracic catheter 3. The thoracic catheter 3 comprises holes 4 enabling to suction air and/or fluid from the patient's body.

As shown in fig. 1 the thoracic catheter 3 is inserted into the chest 100 of a patient. In fig. 1 the trachea 101, the bronchus 102, 103, the left lung 104 and the right lung 106 are shown. The lungs are hereby provided within the pleural section or the pleural space 105. As shown in the present embodiment the right lung 106 cannot re-expand due to air or fluid 107 comprised within the pleural space 105. The air and/or fluid is suctioned through the holes 4 in the thoracic catheter 3, further through the drain tube 2 and collected by the mobile chest drainage unit 1 in the collection chamber 5.

It is to be noted that fig. 1 only shows one example of a mobile chest drainage unit, but the present invention is not limited to the shown type but comprises any other type of chest drainage unit, which mobile, i.e. portable, and which allows to suction air and/or fluid from any cavity or space within the chest.

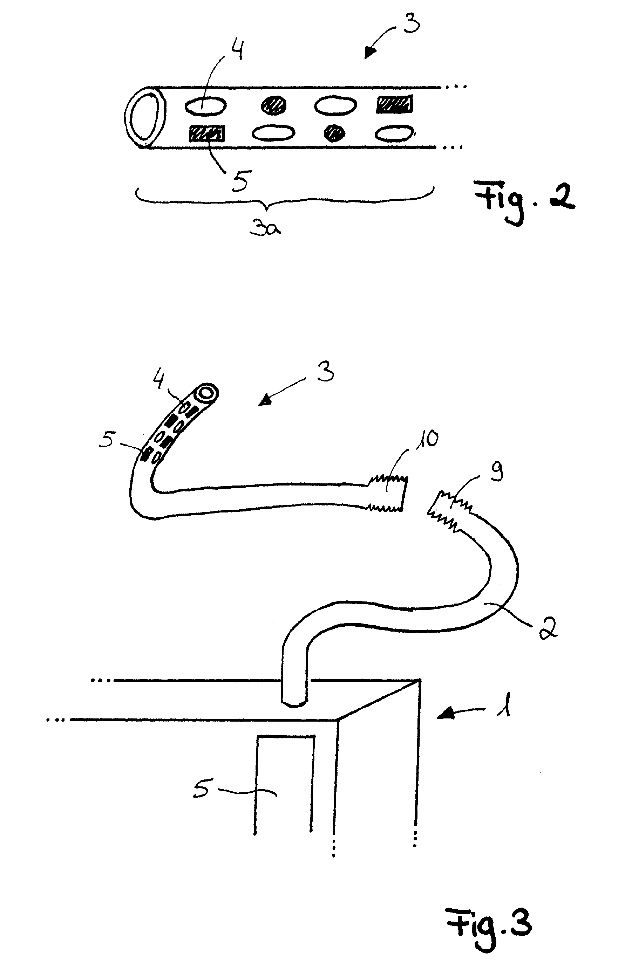

Fig. 2 shows the distal end 3a of a thoracic catheter 3 according to the present invention. The distal end 3a hereby refers to the part of the thoracic catheter 3, which is inserted into the patient's body. According to the present invention, apart from the drainage holes 4 at least one sensor 5 is provided at the distal end 3a of the thoracic catheter 3 for measuring or acquiring information regarding physiological parameters of the patient, such as for example blood pressure, respiration rate, hear beat rate, temperature, pressure within the pleural space and/or other physiological parameters.

Fig. 3 shows a mobile chest drainage unit 1 which can be connected to a thoracic catheter 3 comprising sensors 5 according to the present invention. Fig. 3 hereby shows schematically that the mobile chest drainage unit 1 and the thoracic catheter 3 can be manufactured and provided as separate components connectable to each other.

For the purpose of connection tube connection means 9, 10 are provided for connecting the thoracic catheter 3 to the drain tube 2 of the mobile chest drainage unit 1. Therefore at the thoracic catheter 3 a catheter tube connector 10 is provided and at the drain tube a drain tube connector 9 is provided. In the present example these tube connection means 9, 10 are screw threads engaging with each other, but any other connection is encompassed by the present invention, i. e. any connection which allows to connect the thoracic catheter 3 to the drain tube 2 of the mobile chest drainage unit 1 enabling air and/or fluid to pass through the drain tube 2.

In the following, different embodiments of a mobile chest drainage unit 1 and a thoracic catheter 3 will be explained.

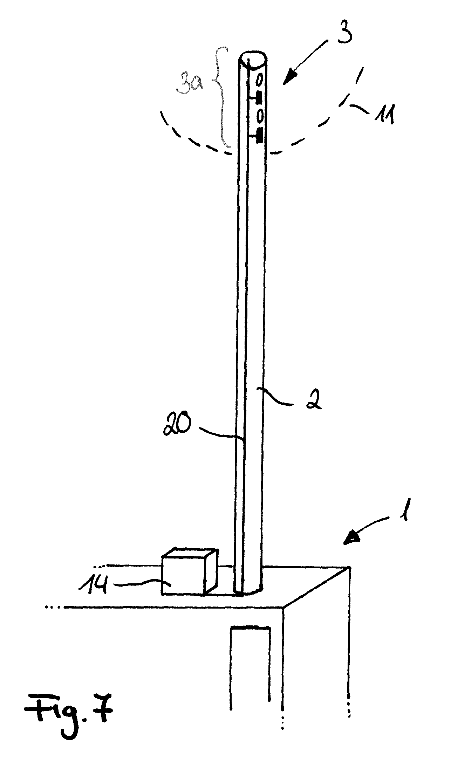

A first possibility as shown in fig, 4 to 6 is to provide the mobile chest drainage unit 1 and the thoracic catheter 3 as separate components which are attachable or connectable to each other. A second possibility shown in fig. 7 is to provide a mobile chest drainage unit 1 and a thoracic catheter 3 as one single unit.

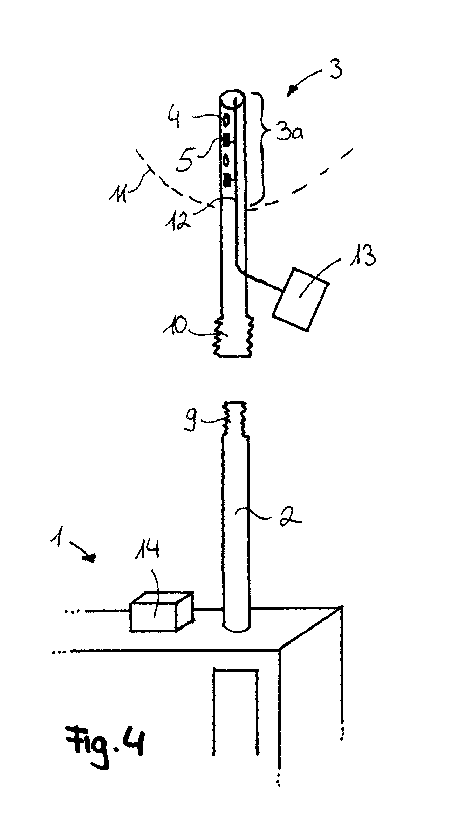

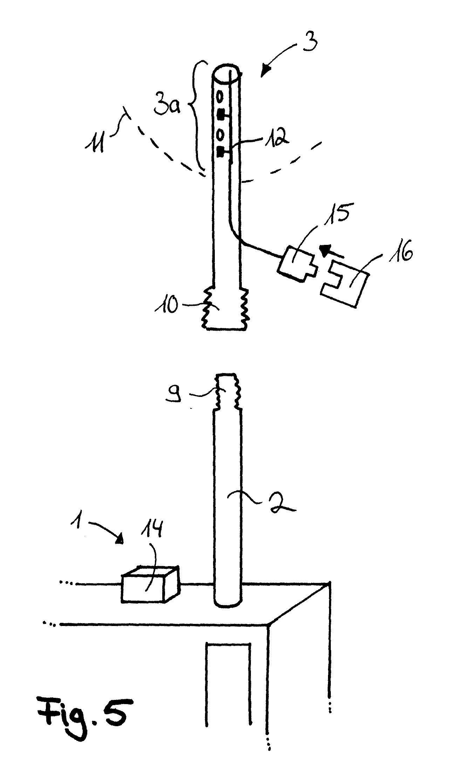

Fig. 4 and 5 show a first embodiment of a mobile chest drainage unit 1. The mobile chest drainage unit 1 comprises a drain tube 2 which has a drain tube connector 9 for connecting the drain tube 2 to the corresponding catheter tube connector 10 of the thoracic catheter 3. Hereby a fluid connection to the thoracic catheter 3 is established, that means a connection which allows air and/or fluid to pass through the drain tube and which prevents the drained air and/or fluid from the drain tube 2.

The mobile chest drainage unit 1 further comprises a receiving means 14 for receiving the information acquired by the at least one sensor 5. In the first embodiment shown in fig. 4 and 5 the receiving means 14 is adapted to wirelessly receive the acquired information, i. e. the receiving means 14 comprises an antenna or any other means enabling the wireless reception of data. The communication from the sensors 5 to the receiving means 14 can hereby be accomplished via radio frequency (RF), bluetooth, infrared (IR) or any other present or future wireless communication technique.

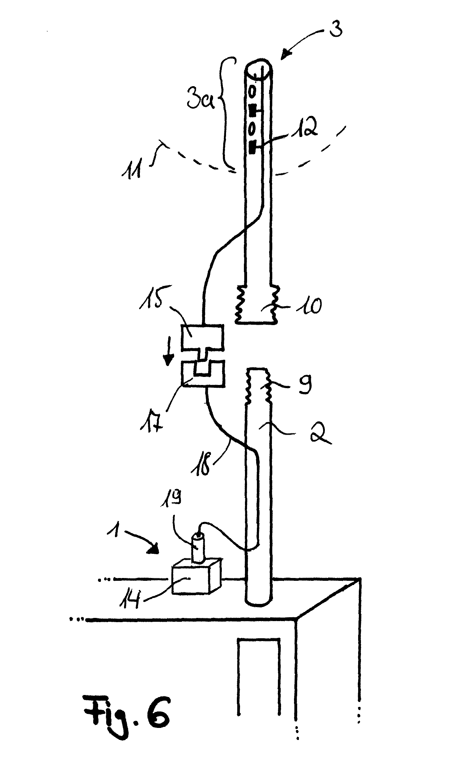

Fig. 6 shows a second embodiment of a mobile chest drainage unit 1 according to the present invention. In this embodiment the mobile chest drainage unit 1 is electronically connectable to the thoracic catheter 3.

In this second embodiment, the drain tube 2 via the drain tube connector 9 is also connectable to the corresponding catheter tube connector 10 of the thoracic catheter 3. Additionally, in this embodiment, the mobile chest drainage unit 1 comprises electronic connection means for electronically connecting the mobile chest drainage unit 1 to the thoracic catheter 3.

The electronic connection means comprise an electric CDU connector 17 which can be electronically connected to an electric catheter connector 15. Since the electric catheter connector 15 is further electronically connected to the at least one sensor 5 of the thoracic catheter 3, it is possible to deliver the acquired information from the sensors 5 to the mobile chest drainage unit 1 via the electronic connection means.

The electric CDU connector 17 and/or the electric catheter connector 15 can be any type of connection adapted to electronically connect the thoracic catheter 3 and the mobile chest drainage unit 1. Specifically, an USB port or any similar electric connection can be provided.

The electronic connection means of the mobile chest drainage unit 1 further comprise a CDU conductor 18, which connects the electric CDU connector 17 with a plug 19, which can be inserted into the receiving means 14 comprised in the mobile chest drainage unit 1. The CDU conductor 18 can hereby at least partly be attached to or embedded into the drain tube 2, so that only the end parts of the CDU conductor are protruding from the drain tube 2.

Thereby an electronic connection between the thoracic catheter 3 and the mobile chest drainage unit 1, specifically between the sensors 5 and the receiving means 14 of the mobile chest drainage unit 1 is accomplished. The receiving means 14 in this case comprises an interface which is adapted to receive the plug 19 and to receive via the electronic connection any information or data submitted from the sensors 5.

Now, different embodiments of a thoracic catheter 3 according to the present invention will be explained. Fig. 4 to 6 show a thoracic catheter 3 which can be inserted into a patient's body. Specifically, the distal end 3a of the thoracic catheter 3 is inserted into the chest of a patient. In the figures, the border 11 of the body of a patient is schematically indicated by a dashed line. The holes 4 and the at least one sensor 5 are provided on the distal end 3a, which during use is inside the body. The thoracic catheter 3 as already explained comprises a catheter tube connector 10 allowing to connect the thoracic catheter 3 to the drain tube of the mobile chest drainage unit 1 by means of a corresponding drain tube connector 10 of the drain tube 2.

A catheter conductor 12 is connected to each sensor 5 for receiving the information acquired by the sensors 5. The catheter conductor 12 connects the sensors to a transmitting means for transmitting the information acquired by the sensors 5 to the mobile chest drainage unit 1. In a preferred embodiment the catheter conductor 12 is a radiopaque line. Such radiopaque line in any type of thoracic catheter 3 has to be provided in order to enable to control the position of the thoracic catheter 3 within the patient's body on a X-ray image. The present invention now proposes to use the already existing radiopaque line for a connection between the sensors 5 and the transmitting means. Thereby, with one single component, two different functions can be provided. With the radiopaque catheter conductor 12 on one hand a radiopaque line necessary for correctly positioning the thoracic catheter 3 is provided and on the other hand an electronic connection between the sensors 5 and the transmitting means can be established.

The catheter conductor 12 hereby can be at least partly attached to or provided within the thoracic catheter 3. At least at the distal end 3a the catheter conductor 12 is fixedly attached to the thoracic catheter 3. At the part, where the thoracic catheter 3 is not inside the body of the patient, the catheter conductor 12 can be protruding from the thoracic catheter 3.

In the first embodiment shown in fig. 4. the catheter conductor 12 leads to a transmitting means, which is a fixed transmitter 14 and adapted to wirelessly transmit the acquired information to the receiving means 14 of the mobile chest drainage unit 1. The wireless transmission can be accomplished according to radiofrequency (RF), Bluetooth, infrared (IR) or any other present or future wireless transmission technique.

In the embodiment shown in fig. 4 the catheter conductor 12 is embedded into the distal end 3a of the thoracic catheter 3, where the thoracic catheter 3 is inserted into the patient's body. The other end of the catheter conductor 12 comprising the fixed transmitter 13 is protruding from the thoracic catheter 3. In an alternative embodiment the complete catheter conductor 12 and the fixed transmitter 13 can also be embedded into the tube of the thoracic catheter 3.

A second embodiment of a thoracic catheter 3 will now be described with reference to fig. 5 and 6. In this case instead of a fixed transmitter 13 connected to the catheter conductor 12, an electric catheter connector 15 can be provided, which can for example be a USB onnector adapted to be plugged into USB ports or the like.

To the electric catheter connector 15 different types of plugs or ports can be connected depending on the actual need. As shown in fig. 5, a connectable transmitter 16 is connected to the catheter connector 15. The connectable transmitter 16 hereby enables to wirelessly transmit the information acquired by the sensors 5 to the receiving means 14 of the mobile chest drainage unit 1. The wireless transmission can be accomplished according to radiofrequency (RF), Bluetooth, infrared (IR) or any other present or future wireless transmission technique.

Fig. 6 shows an alternative use of the second embodiment of a thoracic catheter 3 according to the present invention. In this case instead of a connectable transmitter 16 the electric CDU connector 17 previously described is attached to the electric sensor connector 15. Thereby, an electronic connection is established between the sensors 5 and the receiving means 14, more generally an electronic connection is provided between the thoracic catheter 3 and the mobile chest drainage unit 1.

Now a further embodiment of a mobile chest drainage unit 1 will be explained with reference to fig. 7. This embodiment shows the case where a thoracic catheter 3 is fixedly attached to the mobile chest drainage unit 1 and the mobile chest drainage unit 1 therefore comprises the thoracic catheter 3. In this case a conductor 20 leading from the sensors to the receiving means 14 can be fixedly provided. The conductor can be attached to the drain tube 2 or integrally formed within the drain tube 2. In this embodiment, no connection means have to be provided, neither for the drain tube nor for the electric connection.

The present invention is not limited to the shown embodiments. All components and/or features shown in one embodiment can also be implemented in any other embodiment where appropriate.

## Claims

1. Mobile chest drainage unit (1)
connectable to a thoracic catheter (3) for suctioning air and/or fluid from a patient's body, said thoracic catheter (3) comprising at least one sensor (5) for acquiring information regarding physiological parameters, and
said mobile chest drainage unit (1) comprising a receiving means (14) for receiving the acquired information from the at least one sensor (5).

2. Mobile chest drainage unit (1) according to claim 1,
further comprising a drain tube (2), wherein said drain tube (2) comprises a CDU tube connector (9) for connecting the drain tube (2) to a corresponding catheter tube connector (10) of the thoracic catheter (3).

3. Mobile chest drainage unit (1) according to claim 1 or 2,
wherein said receiving means (14) is adapted to wirelessly receive the acquired information, preferably to receive the acquired information via radiofrequency RF, Bluetooth or infrared IR.

4. Mobile chest drainage unit (1) according to claim 1 or 2,
wherein said receiving means (14) is electronically connectable to the at least one sensor (5) by electronic connection means (17, 18, 19).

5. Mobile chest drainage unit (1) according to claim 4,
wherein said electronic connection means (17, 18, 19) comprise an electric CDU connector (17) electronically connectable to a electric catheter connector (15), which is electronically connected to the at least one sensor (5).

6. Mobile chest drainage unit (1) comprising
a drain tube (2) connectable to a thoracic catheter (3) for suctioning air and/or fluid from a patients body, and
electronic connection means (17, 18, 19) for electronically connecting the mobile chest drainage unit (1) to the thoracic catheter (3).

7. Mobile chest drainage unit (1) according to claim 6,
wherein said drain tube (2) comprises a drain tube connector (9) for connecting the drain tube (2) to a corresponding catheter tube connector (10) of the thoracic catheter.

8. Mobile chest drainage unit (1) according to claim 4,
wherein said electronic connection means (17, 18, 19) comprise an electric CDU connector (17) attached to the drain tube (2) and electronically connectable to a corresponding electric catheter connector (15) of the thoracic catheter.

9. Mobile chest drainage unit (1)
electronically connectable to a thoracic catheter (3).

10. Thoracic catheter (3)
connectable to a mobile chest drainage unit (1),
comprising at least one sensor (5) for acquiring information regarding physiological parameters, and
a transmitting means (13, 15, 16) for transmitting the acquired information to the mobile chest drainage unit (1).

11. Thoracic catheter (3) according to claim 10,
wherein said at least one sensor (5) is provided at the distal end (3a) of the thoracic catheter (3), which is adapted to be inserted into a patient's body.

12. Thoracic catheter (3) according to claim 10 or 11,
wherein said at least one sensor (5) is adapted to acquire information regarding blood pressure, respiration rate, heart beat rate, temperature and/or pressure within the pleural cavity.

13. Thoracic catheter (3) according to any of claims 10 to 12,
wherein said transmitting means (13, 15, 16) via a catheter conductor (12) is electronically connected to the at least one sensor (5).

14. Thoracic catheter (3) according to claim 13,
wherein said catheter conductor (12) is a radiopaque line.

15. Thoracic catheter (3) according to any of claims 10 to 14,
wherein said transmitting means is a fixed transmitter (13) adapted to wirelessly transmit the acquired information, preferably to transmit the acquired information via radiofrequency RF, Bluetooth or infrared IR.

16. Thoracic catheter (3) according to any of claims 10 to 14,
wherein said transmitting means comprises an electric catheter connector (15) electronically connectable to an connectable transmitter (16) adapted to wirelessly transmit the acquired information, preferably to transmit the acquired information via radiofrequency RF, Bluetooth or infrared IR.

17. Thoracic catheter (3) according to any of claims 10 to 14,
wherein said transmitting means comprises an electric catheter connector (15) electronically connectable to a corresponding electric CDU connector (17), which is electronically connected to the mobile chest drainage unit (1).

18. Thoracic catheter (3)
connectable to a mobile chest drainage unit (1) via a drain tube (2) attached to the mobile chest drainage unit (2) and via electronic connection means (17, 18) attached to the drain tube (2) and electronically connected to the mobile chest drainage unit (1).

19. Thoracic catheter (3) according to claim 18,
comprising a catheter tube connector (10) for connecting the thoracic catheter (3) to a corresponding drain tube connector (9) of the drain tube (2).

20. Thoracic catheter (3) according to claim 18 or 19,
further comprising an electronic catheter connector (15) electronically connectable to a corresponding electric CDU connector (17) being electronically connected to the mobile chest drainage unit (1).

21. Thoracic catheter (3) electronically connectable to a mobile chest drainage unit (1).

22. System comprising a mobile chest drainage unit (1) according to any of the preceding claims connected to a thoracic catheter (3) according to any of the preceding claims.

23. Mobile chest drainage unit (1) comprising
a thoracic catheter (3), said thoracic catheter (3) having at least one sensor (5) for acquiring information regarding physiological parameters,
a drain tube (2) connecting the thoracic catheter (3) with the mobile drainage unit (1), and
a conductor (20) along the drain tube (2) for electronically connecting the at least one sensor (5) with the mobile chest drainage unit (1).

24. Method of manufacturing a mobile chest drainage unit (1) according to any of the preceding claims.

25. Method of manufacturing a thoracic catheter (3) according to any of the preceding claims.
